# EUROPEAN PATENT APPLICATION

(11) **EP 4 620 443 A1**
(43) Date of publication of application: **24.09.2025**
(21) Application number: 23891208.3
(22) Date of filing: 03.10.2023
(51) Int. Cl.: A61F 13/15

(54) **INDIVIDUALLY-PACKAGED ABSORBENT ARTICLE, AND PACKAGING SHEET**

(30) Priority: 18.11.2022 JP 2022185094
(71) Applicant: DAIO PAPER CORPORATION, Shikokuchuo-shi Ehime 799-0492 (JP)
(72) Inventor: YONAHA, Susumu, Sakura-shi, Tochigi 329-1411 (JP)
(74) Representative: Epping - Hermann - Fischer
(86) International application number: PCT/JP2023/036043
(87) International publication number: WO 2024/106050

(57) **Abstract**

An individually packaged absorbent article, for which an absorbent article is individually packaged by a packaging sheet made of paper, wherein the packaging sheet is inwardly folded in a longitudinal direction with the absorbent article disposed inside the packaging sheet, and both edge portions in a transverse direction orthogonal to the longitudinal direction are sealed, and the packaging sheet has tensile elongation in the longitudinal direction and tensile elongation in the transverse direction that are 15% or more.

## Description

### TECHNICAL FIELD

The disclosures herein relate to individually packaged absorbent articles and packaging sheets.

### BACKGROUND ART

Generally, absorbent articles such as sanitary napkins, pantyliners, incontinence pads, and the like, are provided as individually packaged absorbent articles (individual packaging) in a state in which they are individually packaged and sealed with packaging sheets for reasons such as hygiene in storage and convenience in carrying.

Resin films and nonwoven fabrics are still main materials of packaging sheets, but paper packaging sheets are also known (e.g., Patent Literature (PTL) 1).

### CITATION LIST

### PATENT LITERATURE

[PTL 1] Japanese Patent Laid-Open Publication No. 2022-24624

### SUMMARY OF THE INVENTION

### PROBLEMS TO BE SOLVED BY THE INVENTION

Packaging sheets made of paper have attracted attention in recent years from viewpoints such as Sustainable Development Goals. However, practical applications of paper packaging sheets have not much advanced compared with resin films and nonwoven fabrics. One of the reasons is that paper tends to tear easily. In order to prevent the paper packaging sheet from tearing, it is conceivable to make the packaging sheet thick, for example, but this may cause the sheet to become hard and compromise the touch of an individually packaged absorbent article.

In view of the above, an aspect of the present invention aims to provide an individually packaged absorbent article using a paper packaging sheet, wherein the packaging sheet is resistant to tearing when opened, and the like, and has a good touch.

### MEANS OF SOLVING THE PROBLEM

An aspect of the present invention is an individually packaged absorbent article, for which an absorbent article is individually packaged by a packaging sheet made of paper, wherein the packaging sheet is inwardly folded in a longitudinal direction with the absorbent article disposed inside the packaging sheet, and both edge portions in a transverse direction orthogonal to the longitudinal direction are sealed, and the packaging sheet has tensile elongation in the longitudinal direction and tensile elongation in the transverse direction that are 15% or more.

### EFFECTS OF THE INVENTION

According to an aspect of the present invention, in the individually packaged absorbent article using a paper packaging sheet, the packaging sheet resistant to tearing upon opening and having a good touch can be provided.

### BRIEF DESCRIPTION OF THE DRAWINGS

[FIG. 1] FIG. 1 is a plan view of an individually packaged absorbent article according to one embodiment of the present invention.
[FIG. 2] FIG. 2 is a plan view of the unfolded individually packaged absorbent article of FIG. 1.
[FIG. 3] FIG. 3 is a cross section along a line I-I of FIG. 1.
[FIG. 4] FIG. 4 is an enlarged view of a portion II of FIG. 1.
[FIG. 5] FIG. 5 is a plan view of an individually packaged absorbent article having a seal according to a variation.
[FIG. 6] FIG. 6 is an enlarged view of a portion III of FIG. 5.
[FIG. 7] FIG. 7 is a drawing describing force exerted on the packaging sheet during opening.
[FIG. 8] FIG. 8 is a drawing describing manufacturing of the individually packaged absorbent article according to one embodiment of the present invention.

### DESCRIPTION OF THE PREFERRED EMBODIMENTS

In the following, embodiments of the present invention will be described with reference to the accompanying drawings. In the drawings, the same or corresponding constituent elements are denoted with the same reference numerals, and description about the elements may be omitted unless particularly described.

### <BASIC STRUCTURE OF INDIVIDUALLY PACKAGED ABSORBENT ARTICLE>

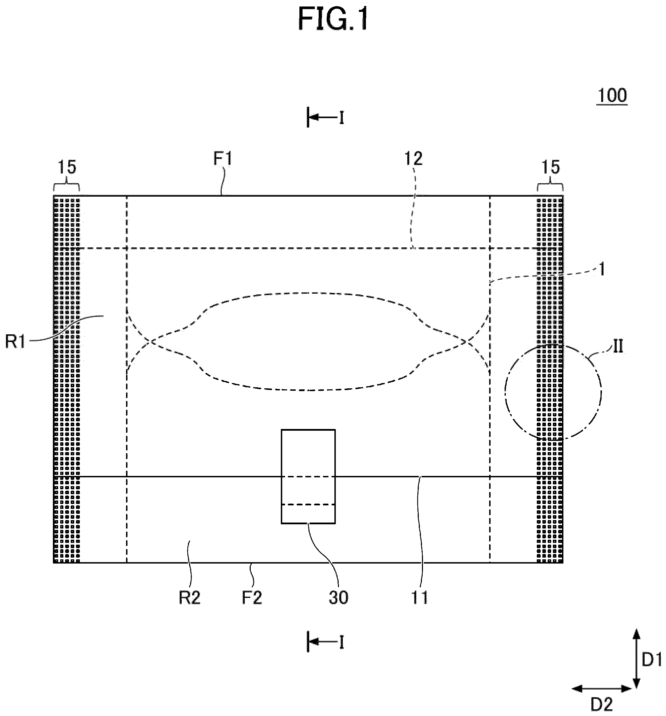

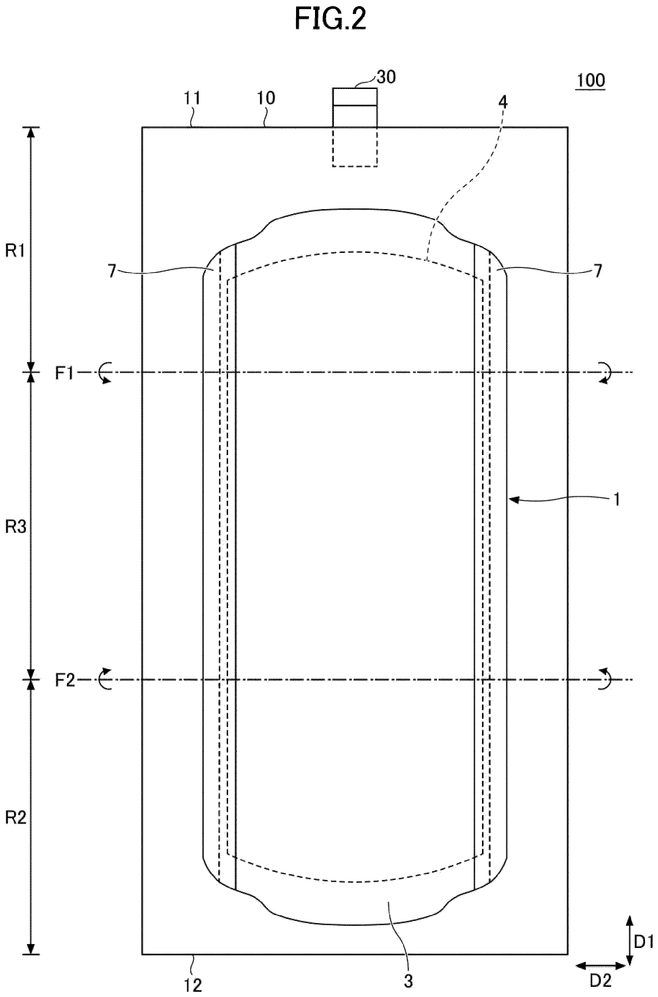

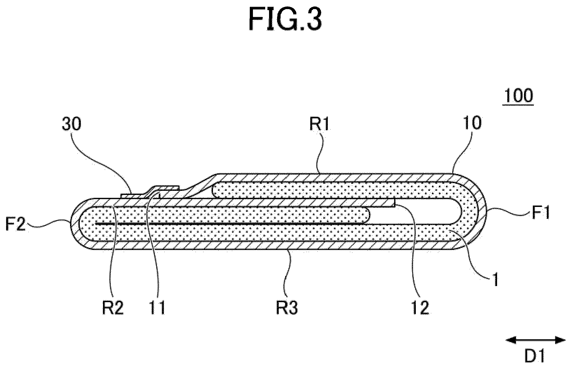

First, a basic structure of an individually packaged absorbent article will be described. FIG. 1 is a plan view of an individually packaged absorbent article 100. FIG. 2 is a plan view of the unfolded individually packaged absorbent article of FIG. 1, which is a drawing viewed from the inside of a packaging sheet 10 or from a surface of an absorbent article 1 facing a skin. FIG. 3 is a cross section along a line I-I of FIG. 1.

As shown in FIGS. 1 to 3, the individually packaged absorbent article 100 includes a packaging sheet 10 and an absorbent article 1 individually packaged by the packaging sheet 10. As shown in FIG. 2, the packaging sheet 10 may have an elongated shape when unfolded, and has a longitudinal direction (vertical direction) D1 and a transverse direction (horizontal direction) D2 perpendicular to the longitudinal direction. The longitudinal direction D1 and the transverse direction D2 of the packaging sheet 10 also correspond to the longitudinal direction and the transverse direction of the absorbent article 1, respectively. In a state of the individually packaged absorbent article 100, the longitudinal direction D1 of the packaging sheet 10 may be referred to as an opening direction and the transverse direction D2 may be referred to as a width direction.

### (ABSORBENT ARTICLE)

The absorbent article 1 packaged by the packaging sheet 10 may be a flat and elongated article to be attached so as to face an outlet of body fluid (menstrual blood, vaginal discharge, urine, etc.). Specific examples of the absorbent article 1 may be sanitary napkins, pantyliners, light incontinence pads, etc. The present description is mainly based on an example in which the absorbent article is a sanitary napkin.

As shown in FIG. 2, for example, the absorbent article 1 may have a liquid-permeable top sheet 3, a liquid-impermeable back sheet (not shown), and an absorber 4 disposed between the top sheet 3 and the back sheet.

For the back sheet, at least a water-resistant sheet material such as an olefin resin sheet, including polyethylene or polypropylene, can be used. A laminated nonwoven fabric in which a nonwoven fabric is laminated on a polyethylene sheet or the like, or a laminated sheet of a nonwoven fabric in which a waterproof film is interposed so as to substantially ensure a liquid impermeability, can be used. In addition, a material having moisture permeability may also be used.

As the top sheet 3, a porous or non-porous nonwoven fabric or a porous plastic sheet or the like is suitably used. As the material fibers constituting the nonwoven fabric, for example, olefins such as polyethylene and polypropylene, synthetic fibers such as polyester and polyamide, regenerated fibers such as rayon and cupra, and mixed fibers of the aforementioned, and natural fibers such as cotton can be used alone or in combination of two or more types.

The absorber 4 is not limited as long as it is a material capable of absorbing and retaining body fluids, but preferably includes a cotton-like pulp and a water-absorbent polymer. Superabsorbent polymer (SAP), superabsorbent fiber (SAF), and a combination of SAP and SAF can be used as the water-absorbent polymer. Examples of pulp include cellulose fibers such as chemical pulp and dissolving pulp obtained from wood, as well as artificial cellulose fibers such as rayon and acetate. The pulp may be hardwood pulp obtained from hardwood, softwood pulp obtained from softwood, or a mixture of the hardwood pulp and the softwood pulp. Moreover, the pulp may be recycled pulp recycled from used pulp.

Thickness of the absorber 4 may be 0.5 to 25 mm. The absorber 4 may be designed with an inflated structure in a region corresponding to the body fluid outlet (body fluid outlet corresponding region) and a region facing a buttock groove behind the body fluid outlet corresponding region. The absorber 4 has a size and shape that do not protrude from the top sheet 3 and back sheet. At the front and rear end edge portions of the absorber, the outer edges of the back sheet and top sheet 3 are bonded using adhesives such as hot melt, or bonding methods such as heat sealing or ultrasonic sealing.

As shown in FIG. 2, on the lateral outside of the absorber 4, side sheets 7, 7 may be provided along the longitudinal direction D1 at both ends of the transverse direction D2. A water-repellent nonwoven fabric or a hydrophilic nonwoven fabric can be used as the side sheet 7.

In the example shown in FIGS. 1 to 3, the absorbent article 1 has no wings, or is what is called a wingless type, but it may be constructed as a winged article having wings extending in the both lateral directions. The wings may be formed by joining the side sheet and the back sheet.

Further, an anti-slip adhesive part to prevent the absorbent article 1 from slipping from underwear when attached may be provided on a surface facing the back sheet (a non-skin surface, or the surface facing the underwear when attached) of the absorbent article 1. The anti-slip adhesive part may be formed in a plurality of strips extending in the longitudinal direction D1 or the transverse direction D2.

The total length of the absorbent article 1 may be 140 to 430 mm, and the width of the absorbent article 1 (when having wings, the width of the body excluding the wings) may be 40 to 130 mm.

### (PACKAGING SHEET)

The packaging sheet 10 in the present embodiment is made of paper. Using a paper packaging sheet 10 can contribute to reduction of plastics and achievement of Sustainable Development Goals. In addition, paper can give a unique texture, for example, an appearance and touch of a gentle impression of a natural material. In the present description, paper refers to vegetable fibers or other fibers bonded with a binding agent to form a thin, flat sheet. In particular, paper made of vegetable fibers as a main raw material, for example, can refer to those containing 50% or more of vegetable fibers, especially cellulose fibers, preferably 80% or more. The vegetable fibers (pulp) used as the raw material of paper may include wood pulp, non-wood pulp, and waste paper pulp, and may be either mechanical pulp or chemical pulp. The pulp may be pulp recycled from components of absorbent articles and/or packaging sheets for absorbent articles. Further, additives may be added to the paper. Examples of paper include various types of paper such as Western paper, Japanese paper, converted paper, and synthetic paper. Furthermore, paper conventionally used for other purposes, such as newsprint paper, printing paper (including wood free paper), writing paper, drawing paper, packaging paper, thin paper, hybrid paper, and the like may be used. In the case of thin paper, thin Japanese vellum, Indian paper, rice paper, glassine paper, tissue paper, toilet paper, filter paper, and the like may be used.

In the present description, a term "paper packaging sheet" mainly refers to a sheet containing the above-mentioned paper. The paper packaging sheet may include not only a packaging sheet made of only paper, but also a laminated sheet in which paper and a sheet made of a material other than paper are laminated. When the packaging sheet 10 includes a sheet made of a material other than paper, the material other than paper may be a resin film, nonwoven fabric, or the like. However, when the packaging sheet 10 is made of only paper, it is particularly preferable from the viewpoint of reducing plastic and/or giving a natural texture unique to paper to the product.

The paper packaging sheet 10 may be subjected to some processing. This processing may include, for example, crepe finishing, embossing, calendering, water-repellent finishing, slit processing, ply processing, printing processing, and the like. Strength and/or flexibility of the paper can be improved by crepe finishing or embossing. An antifouling function can be enhanced by water-repellent finishing (e.g., a water-repellent containing a silicone resin, a paraffin resin, a fluororesin, or the like is applied). In addition, when water-repellent finishing is performed on the back surface of the absorbent article 1, which is opposite to the anti-slip adhesive part, the anti-slip adhesive part can be brought into contact with the packaging sheet 10 without a release sheet.

The size of the packaging sheet 10 depend on the size and shape of the absorbent article 1 to be packaged. For example, when the packaging sheet 10 is fully spread out (not folded back), the length of the longitudinal direction D1 (sometimes referred to simply as length) can be set to 100 to 450 mm, and the length of the transverse direction D2 (sometimes referred to simply as width) can be set to 70 to 250 mm. In the illustrated example, the packaging sheet 10 has a rectangular shape when it is unfolded, but may have a shape such as an oblong shape, for example.

### <PACKAGING STRUCTURE OF INDIVIDUALLY PACKAGED ABSORBENT ARTICLE>

As shown in FIGS. 1 to 3, the absorbent article 1 is folded and packaged with the packaging sheet 10 to form an individually packaged absorbent article 100. At the time of folding, as shown in FIG. 2, the absorbent article 1 is placed on the inner surface of the packaging sheet 10 so that the back sheet of the absorbent article 1 faces the inner surface of the packaging sheet 10. Both the packaging sheet 10 and the absorbent article 1 are folded in the longitudinal direction D1 at a first folding line F1 and a second folding line F2 along the width direction D2. More specifically, at the first folding line F1, a first region R1 including one end 11 of the longitudinal direction D1 of the packaging sheet 10 is folded back in the longitudinal direction D1, and at the second folding line F2, a second region R2 including the other end 12 of the longitudinal direction D1 of the packaging sheet 10 is folded back. The region between the first region R1 and the second region R2 of the packaging sheet 10 is a third region R3. In the illustrated example, the packaging sheet 10 is folded so that the second region R2 is folded first and the first region R1 overlaps the outer surface of the second region R2 (FIGS. 1 and 2), but the folding order of the first region R1 and the second region R2 may be reversed. After packaging the absorbent article 1 by inwardly tri-folding the packaging sheet 10 (folding the packaging sheet 10 in three) with the absorbent article 1, both edge portions of the transverse direction D2 are sealed along the longitudinal direction D1, and seal parts 15, 15 are formed.

Such a packaging form folded in three or more can be relatively easily formed, and the absorbent article 1 can be packaged hygienically, and the absorbent article 1 can be easily removed. The folding form is not limited to folding in three; it can also be folded in four or more, or in two.

As shown in FIG. 1, in the individually packaged absorbent article 100, both edges of the transverse direction D2 where the absorbent article 1 is not present are sealed, and the seal parts 15, 15 are formed. The seal parts 15, 15 may be formed over the entire length of the longitudinal direction D1 of the individually packaged absorbent article 100. This is preferable from the viewpoint that it is possible to prevent dirt, dust, fingers or small objects from entering from the end edge in the transverse direction (sealing property is obtained). The seal parts 15, 15 may be formed by, for example, compression bonding the packaging sheets 10 in the thickness direction by inserting into a pair of rolls and applying pressure by both rolls (described later).

A range of the transverse direction D2 provided with the seal part 15 may be within a range of 20 mm from the end edge of the transverse direction D2. The seal part 15 may be formed in the entire range, or in a part of the range, for example, at a position away from the end edge of the transverse direction D2. The length (width) of the transverse direction D2 of the seal part 15 is preferably 3 to 15 mm.

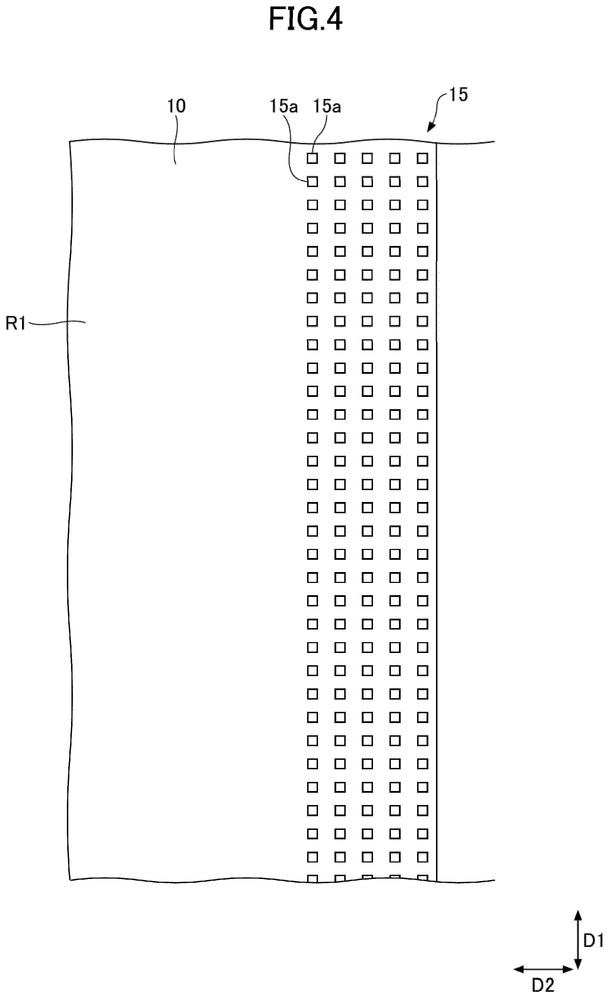

FIG. 4 is an enlarged view of a portion II including the seal part 15. As shown in FIG. 4, the seal part 15 may include a plurality of compression bonded parts (or joints) 15a, 15a, ... which are spaced apart from each other in planar view. The respective compression bonded parts 15a, 15a, ... are portions where the packaging sheets 10 are compression bonded in the thickness direction. In the illustrated example, portions other than the plurality of compression bonded parts 15a, 15a, ... are non-joined parts where the packaging sheets 10 are not joined to each other. Thus, since the compression bonded parts 15a, 15a, ... are interspersed apart from each other, it is possible to easily peel off the packaging sheets during opening and improve opening ease compared with, for example, the case where the packaging sheets 10 are continuously bonded to each other over the entire surface of the seal part 15, and it is also possible to prevent the opposite edge portions of the individually packaged absorbent article 100 from becoming excessively hard and compromising the touch.

The plan view shape of each one of the compression bonded parts 15a, 15a, ... is a square in the example shown in FIG. 4, but it is not limited to the illustrated shape shown, and it may be, for example, a quadrilateral other than a square such as a rectangle, a parallelogram, a polygon other than a quadrilateral, a circle, an oval, a heart, a star, a drop, and the like. In the seal part 15, the size of one of the compression bonded parts 15a may be a square having sides of 0.2 to 2.5 mm or a size having an area equivalent to the aforementioned square. Moreover, arrangement of the plurality of compression bonded parts 15a, 15a, ... may be in a grid pattern as shown in FIG. 4, or a staggered pattern.

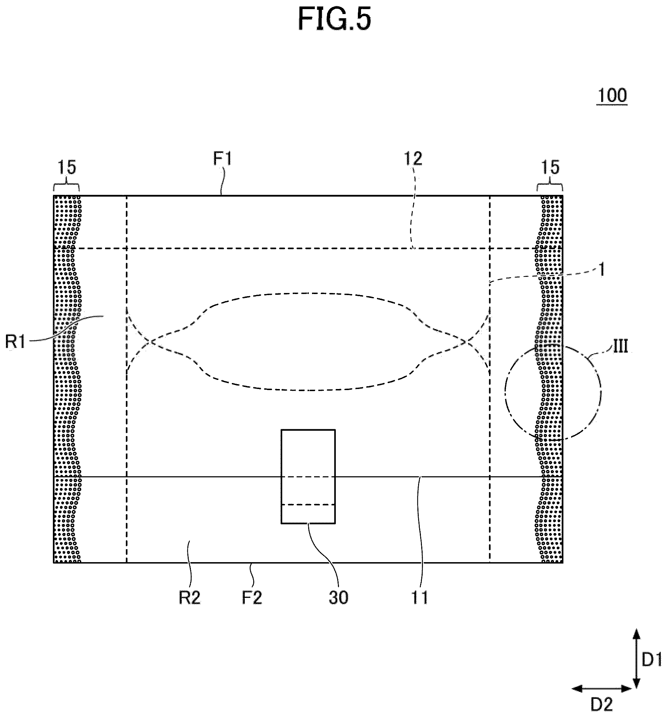

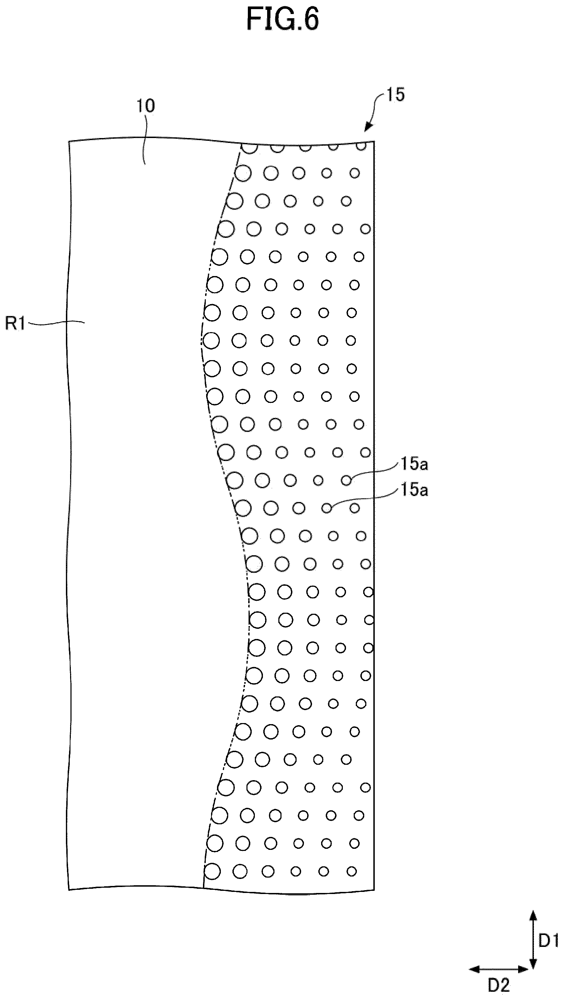

Configurations of the seal part 15 and the compression bonded part 15a are not limited to the example shown in FIGS. 1 and 4. FIG. 5 shows an example of an individually packaged absorbent article 100 in which the configuration of the seal part 15 differs from that of FIG. 1, and FIG. 6 shows an enlarged view of portion III of FIG. 5. In the examples shown in FIGS. 1 and 4, the inner edge of the seal part 15 in the transverse direction D2 extends linearly along the longitudinal direction D1, but may extend in a curved shape, for example, in a wavy shape, as shown in FIGS. 5 and 6. In the examples shown in FIGS. 1 and 4, the size and shape of one of the compression bonded parts 15a are all the same, but as shown in FIGS. 5 and 6, the size of one of the compression bonded parts 15a may increase toward the inner side of the transverse direction D2. This arrangement of the compression bonded parts 15a, 15a, ... can enhance bonding strength of the compression bonded part 15a at the position inside the transverse direction D2, which is a starting position of peeling, and is preferable in that it can prevent unintended opening of the individually packaged absorbent article 100 before use.

As shown in FIG. 1, the individually packaged absorbent article 100 may be provided with a fastening tape 30 near the end edge of the first region R1 (one end 11 of the packaging sheet 10) and at the center of the transverse direction D2. The fastening tape 30 may be provided over the first region R1 and the second region R2 so as to straddle one end 11 of the packaging sheet 10, as shown in FIG. 1. With the fastening tape 30 provided, the user can lift and pull the first region R1 with the fastening tape 30 when opening the individually packaged absorbent article 100, making opening easier. In addition, with the fastening tape 30, when replacing the absorbent article, a used absorbent article can be wrapped with the packaging sheet obtained from a newly opened individually packaged absorbent article, and then the end of the packaging sheet can be fastened to another part of the packaging sheet. This prevents the packaging sheet from opening and exposing the used absorbent article, and the used absorbent article can be disposed of hygienically.

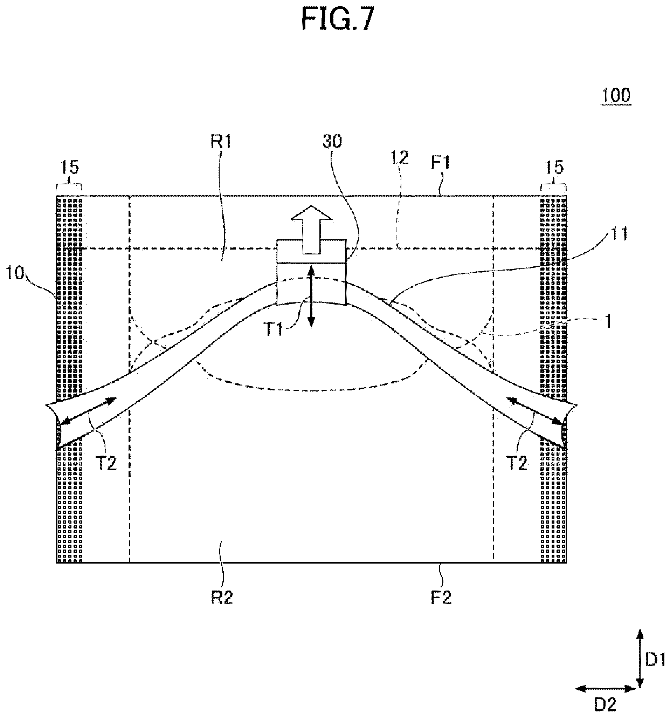

FIG. 7 shows a state of the individually packaged absorbent article 100 during opening. For example, the user can hold the fastening tape 30 with one hand and the second folding line F2 with the other hand and pull the packaging sheet 10 in the opposite direction in the longitudinal direction D1. As a result, in the seal parts 15, 15, the first region R1 is gradually peeled off from the second region R2 joined directly under the first region R1. At this time, the packaging sheet 10 is likely to be subjected to a tensile force in the vicinity of the seal parts 15 and 15 along the transverse direction D2 of the packaging sheet 10 itself. That is, the packaging sheet 10 in the region where the seal parts 15, 15 are formed and the vicinity thereof is likely to be subjected to a tensile force in the directions T2, T2 (FIG. 7). When the fastening tape 30 is provided, the packaging sheet 10 itself is likely to be subjected to a tensile force in the longitudinal direction D1 in the region where the fastening tape 30 is adhered and the vicinity thereof. That is, the packaging sheet 10 in the region where the fastening tape 30 is adhered and the vicinity thereof is likely to be subjected to a tensile force in the direction T1 (FIG. 7). At the time of opening, the packaging sheet 10 is likely to be torn by the above-described tensile force. However, since the direction in which the packaging sheet 10 is pulled during opening varies depending on how the user opens it, specifically, which part of the individually packaged absorbent article 100 is held, how force is applied, and the like, the packaging sheet 10 may be subjected to forces in various directions.

In the present embodiment, the packaging sheet 10 is used such that the tensile elongation (E1) in the longitudinal direction (opening direction in the individually packaged absorbent article 100) D1 and the tensile elongation (E2) in the transverse direction (width direction in the individually packaged absorbent article 100) D2 are 15% or more. Furthermore, it is preferable to use the packaging sheet 10 such that the tensile elongation (E1) in the longitudinal direction (opening direction in the individually packaged absorbent article 100) D1 of the packaging sheet 10 is not less than 15% and the tensile elongation (E2) in the transverse direction (width direction in the individually packaged absorbent article 100) D2 is not less than 20%. The tensile elongation (%) (simply referred to as elongation) is an elongation percentage at a maximum load measured using a tensile testing machine such as a Tensilon testing machine (made by Orientec Co., Ltd.), and is a ratio of the elongation amount (difference between the length at the maximum load and the initial length) when the initial length is 100%. Since the packaging sheet 10 has the tensile elongation as described above, the applied tensile force can be absorbed by the elongation of the material in both the longitudinal direction D1 and the transverse direction D2, so that the packaging sheet 10 becomes resistant to tearing. Further, both the tensile elongation (E1) in the longitudinal direction (opening direction in the individually packaged absorbent article 100) D1, and the tensile elongation (E2) in the transverse direction (width direction in the individually packaged absorbent article 100) of the packaging sheet 10 may be 45% or less. Moreover, in the packaging sheet 10, it is preferable that the tensile elongation (E1) in the longitudinal direction D1 is 25% or less, and the tensile elongation (E2) in the transverse direction D2 is 45% or less. Thus, a feeling of using paper can be prevented from being compromised.

In order to prevent the packaging sheet from being torn, it is conceivable to use a thick or rigid material as the packaging sheet, but in this case, the flexibility of the packaging sheet may be lowered and the touch of the packaging sheet may be compromised. By using a packaging sheet with relatively high tensile elongation as in the present embodiment, the paper packaging sheet can become resistant to tearing without using a thick or rigid material. Therefore, it is possible to provide an individually packaged absorbent article that maintains the touch of the packaging sheet and is resistant to tearing upon opening (with good opening ease).

To increase the tensile elongation of the packaging sheet 10, crepe paper with crepe finishing can be used as the packaging sheet 10, for example. In the crepe finishing, it is preferable that an extending direction of wrinkles formed by the crepe finishing is in the longitudinal direction (opening direction in the individually packaged absorbent article) D1 of the packaging sheet 10, and the direction in which the wrinkles line up is in the transverse direction (width direction in the individually packaged absorbent article) D2 of the packaging sheet 10. When the packaging sheet 10 is crepe paper, the crepe ratio may be 8 to 25%. The crepe paper can improve the tensile elongation and tensile strength of the packaging sheet 10 even if the thickness of the material is small. In addition, since the crepe finishing makes a moderately fine protrusions and recesses on the surface, the touch and ease of holding are also improved.

When comparing the tensile elongation (E1) of the packaging sheet 10 in the longitudinal direction D1 and the tensile elongation (E2) in the transverse direction D2, a value obtained by dividing the larger tensile elongation by the smaller tensile elongation (E2/E1 or E1/E2) is preferably 1 to 2 and more preferably 1.1 to 1.8. The value obtained by dividing the larger tensile elongation by the smaller tensile elongation is equal to or larger than 1, and is expressed as E2/E1 in the case of E1 < E2, E1/E2 in the case of E1 > E2, and 1 in the case of E1 = E2. If the difference due to the tensile elongation depending on the direction becomes excessively large depending on the range of the above values, a stress when force is applied in a specific direction increases, and can actually lead to tear. However, the difference in resistance to tearing depending on the direction can be reduced by the value of the above ratio. Therefore, the packaging sheet 10 can be satisfactorily prevented from being torn regardless of the direction of the force applied by the way the user opens the packaging. In addition, the variation in the flexibility of the packaging sheet 10 depending on the direction of tension can be reduced, and the packaging sheet 10 can be provided with a comfortable use.

The tensile elongation (E2) in the transverse direction D2 of the packaging sheet 10 may be larger than the tensile elongation (E1) in the longitudinal direction D1. Furthermore, it is preferable that the value (E2/E1) obtained by dividing the tensile elongation (E2) in the transverse direction D2 of the packaging sheet 10 by the tensile elongation (E1) in the longitudinal direction D1 is 1 to 2 or 1.1 to 1.8. This configuration is preferable on the point of preventing tearing due to tension (tension extending the packaging sheet 10 along T2, which is a direction T2 in FIG. 7) applied to the region where the seal parts 15, 15 are formed on both edge portions of the transverse direction D2 and the vicinity.

Furthermore, it is preferable that the tensile strength (S1) in the longitudinal direction D1 (in the opening direction of the individually packaged absorbent article 100) of the packaging sheet 10 is 3.5 N/25 mm or more and 28 N/25 mm or less. Furthermore, it is preferable that the tensile strength (S2) in the transverse direction D2 (in the width direction of the individually packaged absorbent article 100) is 15 N/25 mm or more and 35 N/25 mm or less. Tensile strength can also be measured using a tensile testing machine. If the tensile strength is within the above range, the effect that the packaging sheet 10 becomes resistant to tearing upon opening can be further improved.

When the tensile strength (S1) of the packaging sheet 10 in the longitudinal direction D1 is compared with the tensile strength (S2) of the transverse direction D2, the value obtained by dividing the larger tensile strength by the smaller tensile strength (S2/S1 or S1/S2) may preferably be 2 to 4, and more preferably 3 to 5. The value obtained by dividing the larger tensile strength by the smaller tensile strength is equal to or greater than 1, and is expressed as S2/S1 when S1 < S2, S1/S2 when S1 > S2, and 1 when S1 = S2. With the above range of values, the strength of the longitudinal direction D1 and the transverse direction D2 can be balanced to some extent, and the difference in the resistance to tearing depending on the direction can be reduced.

The tensile strength (S2) in the transverse direction D2 of the packaging sheet 10 may be larger than the tensile strength (S1) of the longitudinal direction D1. Furthermore, it is preferable that the value (S2/S1) obtained by dividing the tensile strength (S2) of the transverse direction D2 of the packaging sheet 10 by the tensile strength (S1) in the longitudinal direction D1 is 2 to 4, or 3 to 5. This configuration is preferable on the point of preventing tearing due to tension (tension extending the packaging sheet 10 along T2, which is a direction T2 in FIG. 7) applied to the region where the seal parts 15, 15 are formed on both edge portions of the transverse direction D2 and the vicinity. When the packaging sheet 10 is torn, occurrence of a tear along the longitudinal direction D1 tends to be avoided. This enables prevention of the packaging sheet 10 from being compromised by actions such as lifting the packaging sheet 10 in the longitudinal direction D1 when the packaging sheet 10 is unfolded during opening or when wrapping the used absorbent article during replacement of the absorbent article.

In order to increase the tensile elongation and/or tensile strength of the packaging sheet 10, the fiber diameter and/or fiber length of the constituent fibers of the paper may be increased. In addition, a paper strength enhancer such as starch may be added. In order to increase the tensile elongation in particular, the constituent fibers may contain stretchable fibers, but it is preferable not to include stretchable fibers from the viewpoint of achieving Sustainable Development Goals.

The thickness of the paper packaging sheet 10 (in the case of crepe paper, the thickness after crepe finishing) may preferably be 40 to 280 µm, more preferably 70 to 150 µm. Weight of the packaging sheet 10 may preferably be 12 to 50 g/m², more preferably 15 to 35 g/m². Such a relatively thin packaging sheet and/or a small weight of the packaging sheet may be flexible, so it is comfortable to touch, and noise generated during opening and carrying can be reduced. According to the present embodiment, even when such a paper packaging sheet having a thin thickness and/or a small weight is used, it is possible to provide an individually packaged absorbent article having a packaging sheet 10 that is resistant to tearing upon opening.

### <MANUFACTURING OF INDIVIDUALLY PACKAGED ABSORBENT ARTICLE>

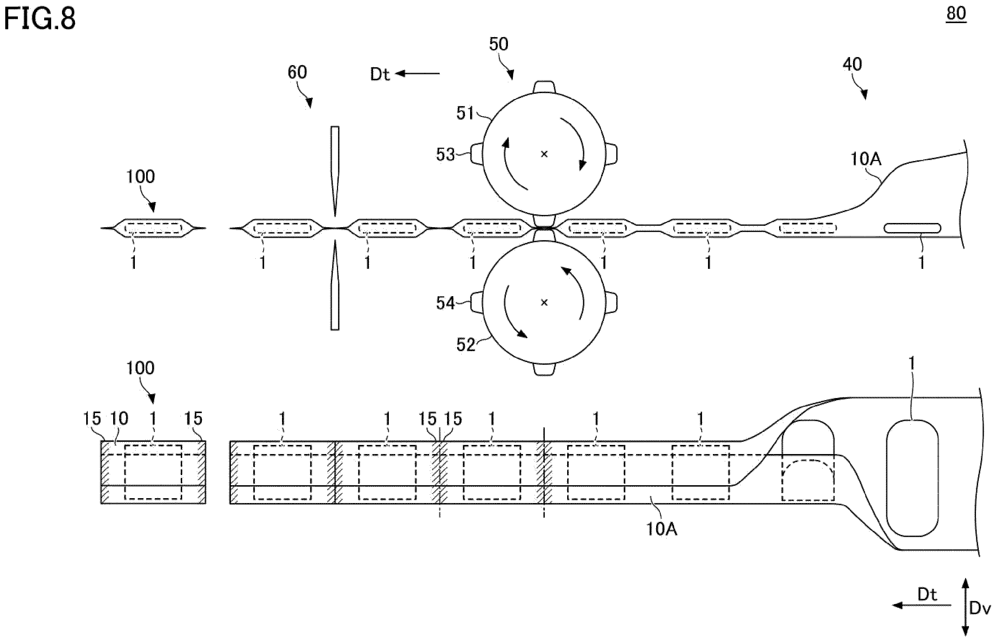

FIG. 8 shows a schematic diagram of an example of a manufacturing apparatus 80 of an individually packaged absorbent article 100. As shown in FIG. 8, a packaging sheet long body 10A to be a paper packaging sheet 10 (FIGS. 1 to 3) is transported in a conveyance direction Dt. The packaging sheet long body 10A can be obtained in a rolled-up state, and can be used by unrolling it from the roll. The packaging sheet long body 10A is transported along its longitudinal direction, and the absorbent articles 1, 1 are disposed on the inner surface of the packaging sheet long body 10A at intervals. The absorbent article 1 is disposed so that the longitudinal direction of the absorbent article 1 is along an orthogonal direction Dv orthogonal to the conveyance direction Dt. Subsequently, the packaging part 40 of the manufacturing apparatus 80 folds the packaging sheet long body 10A with the absorbent article 1 in the orthogonal direction Dv (i.e., the longitudinal direction D1 of the absorbent article 1) at a folding position along the conveyance direction Dt. It is preferable that the absorbent article 1 is folded in three so that the absorbent article 1 is not exposed after folding.

The absorbent articles 1, 1, ... disposed at intervals in the conveyance direction Dt are further conveyed to the seal forming part 50 of the apparatus 80 in a state of being packaged by the packaging sheet long body 10A. As shown in FIG. 8, the seal forming part 50 includes a pair of rolls 51, 52. In the seal forming part 50, at a position between the absorbent articles 1, 1, ... (a position where the absorbent article 1 is not disposed), the packaging sheet long bodies 10A stacked in the thickness direction are pressed in the thickness direction by the pair of rolls 51, 52 to form the seal part. In the seal forming part 50, the seal parts 15, 15 (e.g., the seal part on the right of the individually packaged absorbent article downstream in the conveyance direction and the seal part on the left of the individually packaged absorbent article upstream in the conveyance direction) of two adjacent individually packaged absorbent articles are simultaneously formed.

Of the pair of rolls in the seal forming part 50, the first roll 51 may be provided with pressurizing parts 53, 53, ... along the circumferential direction, and similarly, the second roll 52 may be provided with pressurizing parts 54, 54, ... along the circumferential direction. Here, for example, the surfaces of each of the pressurizing parts 53, 53, ... of the first roll 51 may be provided with a plurality of protrusions that later correspond to the compression bonded parts 15a, 15a, ... (FIG. 4, etc.). Conversely, the surfaces of the pressurizing parts 54, 54, ... of the second roll 52 may be flat. By rotating the pair of rolls as above, the pressurizing part 53 of the first roll 51 and the pressurizing part 54 of the second roll 52 face each other, and the packaging sheet long body 10A sandwiched between the rolls is pressurized in the thickness direction. At this time, the temperature of the roll may be raised so that the packaging sheet long body 10A is heated.

After the seal part is formed in the seal forming part 50, the packaging sheet long body 10A is cut at the center of the conveyance direction Dt in the region where the seal part is formed by a cutting part 60, so that the individually packaged absorbent article 100 having the seal parts 15 and 15 at the edge portion of the conveyance direction Dt can be obtained. The seal forming part 50 and the cutting part 60 may be integrated means, that is, forming and cutting of the seal part may be performed by a single apparatus.

The conveyance direction Dt may be in a flow direction (MD direction or vertical line direction) during manufacturing the packaging sheet long body 10A, and the orthogonal direction Dv may be in the transverse direction (CD direction or horizontal line direction) orthogonal to the flow direction (MD direction). That is, the transverse direction D2 of the packaging sheet 10 in the individually packaged absorbent article 100 may be in the MD direction, and the longitudinal direction D1 of the packaging sheet 10 may be in the CD direction. However, the MD direction and the CD direction of the material in the individually packaged absorbent article 100 of the packaging sheet 10 are not limited to the above, and the transverse direction D2 of the packaging sheet 10 in the individually packaged absorbent article 100 may be in the CD direction, and the longitudinal direction D1 of the packaging sheet 10 may be in the MD direction.

### EXAMPLES

### (EXAMPLE 1)

A packaging sheet (length in opening direction D1: 195 mm × length in width direction D2: 120 mm) was prepared. The packaging sheet was made of crepe paper (thickness: 80 µm, weight: 18 g/m²) produced mainly from hardwood pulp and softwood pulp. A sanitary napkin (20.5 cm, wingless) was disposed on the inner surface of the packaging sheet so that the length of the sanitary napkin was aligned with the length of the packaging sheet. Then, the packaging sheet and the sanitary napkin were folded in three as shown in FIGS. 1 to 3, and both edge portions in the transverse direction of the packaging sheet were compression bonded by a pair of rolls to form a seal part dotted with a plurality of compression bonded parts as shown in FIGS. 1 and **4****.** A plurality of dot-shaped protrusions corresponding to the compression bonded parts were formed on one surface of the pair of rolls, and the other surface was flat. The compression bonding was performed at a line speed of 1000 sheets per minute and at room temperature.

Both tensile elongation and tensile strength of the packaging sheet used in Example 1 were measured. Seal strength of the seal part of the individually packaged absorbent article was also measured for reference. In addition, the obtained opening ease of the individually packaged absorbent article was evaluated.

### (COMPARATIVE EXAMPLE 1)

A commercial product of an individually packaged absorbent article manufactured using a paper packaging sheet was prepared. Both tensile elongation and tensile strength of the packaging sheet used in Comparative Example 1 were measured. Seal strength of the seal part of the individually packaged absorbent article was also measured for reference. In addition, the obtained opening ease of the individually packaged absorbent article was evaluated.

Measurement and evaluation methods were as follows. The results are shown in Table 1:

### <TENSILE ELONGATION AND TENSILE STRENGTH>

In order to measure characteristics of the packaging sheet in the longitudinal direction (opening direction in the individually packaged absorbent article), the packaging sheet was cut into a rectangular test piece having 120 mm in the longitudinal direction of the packaging sheet (opening direction in the individually packaged absorbent article) × 25 mm in the transverse direction of the packaging sheet (opening direction in the individually packaged absorbent article). A tensile test was conducted using a tensile testing machine (Tensilon testing machine "RTC1210", produced by Orientec Co., Ltd.) at a chuck interval of 50 mm and a tensile speed of 500 mm/min, and the tensile elongation (%) and tensile strength (N) were measured.

In order to measure characteristics of the packaging sheet in the transverse direction (width direction in the individually packaged absorbent article), the packaging sheet was cut into a rectangular test piece having 120 mm in the transverse direction of the packaging sheet (width direction in the individually packaged absorbent article) × 25 mm in the longitudinal direction of the packaging sheet (opening direction in an individually packaged absorbent article). The same test as above was conducted and the tensile elongation (%) and tensile strength (N) were measured.

### <SEAL STRENGTH>

In each example, the edge portion of the individually packaged absorbent article in the longitudinal direction (including the seal part having a width of 6 mm) was cut at a position of 25 mm from the end edge in the transverse direction of the packaging sheet, and a test was conducted using the above-mentioned tensile testing machine used for measuring the tensile elongation and tensile strength. Specifically, a portion of the exposed longitudinal end edge of the packaging sheet (one longitudinal end 11 included in the first region R1 in FIG. 1) was slightly peeled off, one chuck was attached to the portion which was peeled off, and the other chuck was attached near the longitudinal end edge of the individually packaged absorbent article (folding line F2 in FIG. 1). Both of the chucks were pulled in opposite directions with a chuck interval of 10 mm and a tensile speed of 100 mm/min. The maximum point load (N) was set as the seal strength (peeling strength).

In all the above measurements of tensile elongation, tensile strength, and seal strength, an average value of five measurements was adopted.

### <OPENING EASE>

The individually packaged absorbent article obtained in each example was opened in a general manner. Specifically, the packaging was unfolded in the longitudinal direction by holding the exposed longitudinal end edge (one end 11 in the longitudinal direction included in the first region R1 in FIG. 1) near the center in the transverse direction. At this time, it was observed whether the packaging sheet was torn or not. In each example, five individually packaged absorbent articles were opened, and when no packaging sheets were torn, it was designated as "o", and when the packaging sheet was torn in one or more cases, it was designated as "×".

**[Table 1]**

| | Tensile Elongation [%] | | Tensile Strength [N] (Tape Width: 25 mm) | | Seal Strength [N] (Seal Width: 6 mm) | Opening Ease |
|---|---|---|---|---|---|---|
| | Longitudinal Direction (Opening Direction) | Transverse Direction (Width Direction) | Longitudinal Direction (Opening Direction) | Transverse Direction (Width Direction) | | |
| Example 1 | 20.39 | 22.37 | 4.01 | 16.07 | 0.27 | ○ (0 Tears) |
| Comparative Example 1 | 3.37 | 8.51 | 5.65 | 8.49 | 0.21 | × (2 Tears) |

As shown in Table 1, the packaging sheet of Example 1 had a higher tensile elongation than the packaging sheet of Comparative Example 1 in both the longitudinal and the transverse directions (15% or more in longitudinal direction, 20% or more in transverse direction). In Example 1, the packaging sheet with the higher tensile elongation was more resistant to tearing upon opening.

Although the present invention has been described based on the embodiments described above, the present invention is not limited to these embodiments, and various variations and modifications may be made without departing from the scope of the present invention. Moreover, the above-described components can be combined optionally.

Further, specific aspects of the present invention will be described below.

### (Clause 1)

An aspect of Clause 1 is an individually packaged absorbent article, for which an absorbent article is individually packaged by a packaging sheet made of paper, wherein the packaging sheet is inwardly folded in a longitudinal direction with the absorbent article disposed inside the packaging sheet, and both edge portions in a transverse direction orthogonal to the longitudinal direction are sealed, and the packaging sheet has tensile elongation in the longitudinal direction and tensile elongation in the transverse direction that are 15% or more.

According to the aspect of Clause 1, since the packaging sheet has a predetermined tensile elongation, it is possible to provide an individually packaged absorbent article whose packaging sheet is resistant to tearing upon opening. According to the present embodiment, since it is not necessary to use thick or rigid paper for the packaging sheet, the flexibility of the packaging sheet can be ensured, and a product in which a user can feel a good touch when holding the individually packaged absorbent article can be provided.

### (Clause 2)

In an aspect of Clause 2, the tensile elongation in the longitudinal direction is 15% or more, and the tensile elongation in the transverse direction is 20% or more.

According to the aspect of Clause 2, it is possible to further improve the effect that the packaging sheet is resistant to tearing upon opening. During opening, the packaging sheet tends to be subjected to tensile force in the transverse direction in the vicinity of the seal parts formed at both edge portions in the transverse direction. Therefore, by increasing the tensile elongation in the transverse direction in the present embodiment, the packaging sheet resistant to tearing upon opening can be obtained.

### (Clause 3)

In an aspect of Clause 3, the packaging sheet has tensile strength in the longitudinal direction of 3.5 N/25 mm or more and 28 N/25 mm or less, and tensile strength in the transverse direction of 15 N/25 mm or more and 35 N/25 mm or less.

According to the aspect of Clause 3, it is possible to further improve the effect that the packaging sheet is resistant to tearing upon opening.

### (Clause 4)

In an aspect of Clause 4, the packaging sheet is made of crepe paper.

According to the aspect of Clause 4, it is possible to provide the individually packaged absorbent article having the packaging sheet that is more resistant to tearing upon opening and has a better touch.

### (Clause 5)

In an aspect of Clause 5, the packaging sheet is folded so that a first region including one end edge in the longitudinal direction of the packaging sheet overlaps with a second region including another end edge in the longitudinal direction of the packaging sheet, and a fastening tape is provided over the first region and the second region.

According to the aspect according to Clause 5, when the packaging sheet is equipped with fastening tape, it is easier to hold the end of the packaging sheet when opening, which makes the process easier. In addition, the fastening tape has an advantage that it can be used to fasten the packaging sheet after wrapping the used absorbent article when the used absorbent article is to be disposed of. In this configuration, even with the fastening tape, since the packaging sheet has relatively large tensile extension in the present embodiment, the packaging sheet can be prevented from being torn near the fastening tape during opening.

### (Clause 6)

In an aspect of Clause 6, thickness of the packaging sheet is 40 to 280 µm.

As the aspect of Clause 6, by using a relatively thin packaging sheet, softness of the packaging sheet can be ensured and the touch can be improved. In addition, even in such a relatively thin packaging sheet, the packaging sheet has a relatively large tensile elongation according to the present embodiment, so that the packaging sheet can be prevented from being torn during opening.

### (Clause 7)

An aspect of Clause 7 is a packaging sheet made of paper for individually packaging an absorbent article, wherein the packaging sheet is capable of forming an individually packaged absorbent article by being inwardly in a longitudinal direction with the absorbent article disposed inside the packaging sheet, and having both edge portions in a transverse direction orthogonal to the longitudinal direction being sealed, and the packaging sheet has tensile elongation in the longitudinal direction and tensile elongation in the transverse direction of that are 15% or more. Preferably, the tensile elongation of the packaging sheet in the longitudinal direction is 20% or more, and the tensile elongation in the transverse direction is 15% or more.

According to the aspect of Clause 7, it is possible to provide a paper-made packaging sheet for individually packaging an absorbent article which has the same effect as the aspect of Clause 1.

The present application is based on and claims priority to Japanese patent application no. 2022-185094 filed on November 18, 2022, with the Japanese Patent Office, the entire contents of which are hereby incorporated by reference.

### REFERENCE SIGNS LIST

1 absorbent article
3 top sheet
4 absorber
7 side sheet
10 packaging sheet
11 one end in the longitudinal direction
12 the other end in the longitudinal direction
15 seal part
15a compression bonded part
30 fastening tape
40 packaging part
50 seal forming part
60 cutting part
80 individually packaged absorbent article manufacturing apparatus
100 individually packaged absorbent article
D1 longitudinal direction of packaging sheet (opening direction)
D2 transverse direction of packaging sheet (width direction)
F1 first folding line
F2 second folding line
R1 first region
R2 second region
R3 third region

## Claims

1. An individually packaged absorbent article, for which an absorbent article is individually packaged by a packaging sheet made of paper, wherein:
the packaging sheet is inwardly folded in a longitudinal direction with the absorbent article disposed inside the packaging sheet, and both edge portions in a transverse direction orthogonal to the longitudinal direction are sealed; and
the packaging sheet has tensile elongation in the longitudinal direction and tensile elongation in the transverse direction that are 15% or more.

2. The individually packaged absorbent article according to claim 1, wherein the tensile elongation in the longitudinal direction is 15% or more, and the tensile elongation in the transverse direction is 20% or more.

3. The individually packaged absorbent article according to claim 1 or 2, wherein the packaging sheet has tensile strength in the longitudinal direction of 3.5 N/25 mm or more and 28 N/25 mm or less, and tensile strength in the transverse direction of 15 N/25 mm or more and 35 N/25 mm or less.

4. The individually packaged absorbent article according to claim 1 or 2, wherein the packaging sheet is made of crepe paper.

5. The individually packaged absorbent article according to claim 1 or 2, wherein the packaging sheet is folded so that a first region including one end edge in the longitudinal direction of the packaging sheet overlaps with a second region including another end edge in the longitudinal direction of the packaging sheet, and a fastening tape is provided over the first region and the second region.

6. The individually packaged absorbent article according to claim 1 or 2, wherein thickness of the packaging sheet is 40 to 280 µm.

7. A packaging sheet made of paper for individually packaging an absorbent article, wherein:
the packaging sheet is capable of forming an individually packaged absorbent article by being inwardly folded in a longitudinal direction with the absorbent article disposed inside the packaging sheet, and having both edge portions in a transverse direction orthogonal to the longitudinal direction being sealed; and
the packaging sheet has tensile elongation in the longitudinal direction and tensile elongation in the transverse direction that are 15% or more.
